(19) Europäisches Patentamt / European Patent Office / Office européen des brevets

(11) **EP 1 633 849 B1**

(12) **EUROPÄISCHE PATENTSCHRIFT**

(45) Veröffentlichungstag und Bekanntmachung des Hinweises auf die Patenterteilung:
**11.07.2007  Patentblatt 2007/28**

(21) Anmeldenummer: 04730852.3

(22) Anmeldetag: **03.05.2004**

(51) Int Cl.:
*C12M 1/34* (2006.01)          *C12Q 1/02* (2006.01)
*G01N 27/22* (2006.01)

(86) Internationale Anmeldenummer:
**PCT/EP2004/004682**

(87) Internationale Veröffentlichungsnummer:
**WO 2004/096974 (11.11.2004 Gazette 2004/46)**

(54) **VERFAHREN UND VORRICHTUNG ZUR SCHNELLEN BESTIMMUNG EINER BAKTERIELLEN BELASTUNG IN BLUT UND BLUTPRODUKTEN**

METHOD AND DEVICE FOR RAPIDLY DETERMINING A BACTERIAL LOAD IN BLOOD AND BLOOD PRODUCTS

MESURE NON-INVASIVE ET RAPIDE DE LA PRESENCE DE BACTERIES DANS LE SANG ET LES PRODUITS SANGUINS

(84) Benannte Vertragsstaaten:
**AT BE BG CH CY CZ DE DK EE ES FI FR GB GR HU IE IT LI LU MC NL PL PT RO SE SI SK TR**

(30) Priorität: **02.05.2003  DE 3400017
04.11.2003  DE 10351390**

(43) Veröffentlichungstag der Anmeldung:
**15.03.2006  Patentblatt 2006/11**

(73) Patentinhaber:
• **Lmb Lab med Blutbank Technologie GmbH
85445 Schwaig (DE)**
• **Sirotech Ltd
76123 Rehovot (IL)**

(72) Erfinder:
• **PIETERSZ, Ruby,
Sanquin Blood Bank
1006 AC Amsterdam (NL)**
• **FELDBERG, Roman,
c/o Sirotech Ltd.
76123 Rehovot (IL)**

• **JENTSCH, Klaus,
c/o Lmb Lab med Blutbank Technologie GmbH
85445 Schwaig (DE)**
• **SHINAR, Eilat,
c/o Magen David Adom Blood Services
61091 Tel Aviv (IL)**

(74) Vertreter: **Winter, Brandl, Fürniss, Hübner Röss, Kaiser,
Polte Partnerschaft Patent- und Rechtsanwaltskanzlei
Alois-Steinecker-Strasse 22
85354 Freising (DE)**

(56) Entgegenhaltungen:
EP-A- 0 803 728          WO-A-94/03802
DE-A- 2 032 725          DE-A- 19 651 355
US-A- 5 546 006          US-A- 5 583 432

• GOMEZ R ET AL: "Microscale electronic detection of bacterial metabolism" SENSORS AND ACTUATORS B, ELSEVIER SEQUOIA S.A., LAUSANNE, CH, Bd. 86, Nr. 2-3, 20. September 2002 (2002-09-20), Seiten 198-208, XP004380191 ISSN: 0925-4005

EP 1 633 849 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**EP 1 633 849 B1**

**Beschreibung**

1 . Gebiet der Erfindung

Die vorliegende Erfindung bezieht sich im Allgemeinen auf ein Verfahren und eine Vorrichtung zum Überwachen von Mikroorganismenpopulationen, und bezieht sich insbesondere auf ein Verfahren und eine Vorrichtung zum schnellen, nicht-invasiven Testen flüssiger Proben wie etwa Blut und Blutprodukten. Als Blutprodukte kommen z.B. Erythrozyten, Thrombozyten (Blutplättchen), Granulozyten bzw. jeweils deren Konzentrate sowie Plasma und Plasmaderivate oder auch Vollblut in Betracht.

2. Beschreibung des Standes der Technik

Vorrichtungen zum Bestimmen der Mikroorganismenpopulation in einer Probe wie etwa einer Milchprobe oder einer Blutprobe bzw. Blutproduktprobe sind in der Technik wohl bekannt. Unter Verwendung von Näherungen der Wachstumsrate bekannter Bakterienpopulationen werden jeweilige gegenwärtige Populationen in die Vergangenheit extrapoliert. Beispielsweise werden während eines Wachstums von Bakterien in einer Probe Impedanzmessungen an der Probe durchgeführt. Da die Bakterienpopulation in der Probe wächst und einem Stoffwechsel unterliegt, ändert sich die Ionenkonzentration der Probe, und es ändern sich die elektrischen Eigenschaften der Probe, insbesondere seine Impedanz. Durch Messen der Impedanzänderungen bei konstanter Temperatur kann die Anzahl von Mikroorganismen abgeschätzt werden.

Zusätzlich zu unvermeidlichen Fehlern, welche sich aus den oben erwähnten Extrapolation ergeben, sind jedoch existierende Verfahren und Vorrichtungen zum Überwachen von Bakterienpopulationen im Allgemeinen extrem ungenau und erfordern eine lange Brut- und Überwachungsdauer, bevor endgültige Ergebnisse erzielt werden. Dies liegt daran, daß signifikante Änderungen in den elektrischen Eigenschaften der beimpften Probe, welche nicht einer nicht-bakteriellen Aktivität in der Probe zugeordnet werden können, nur bei hohen Konzentrationen von Mikroorganismen auftreten. Nachdem längere Brutdauern erforderlich sind, um hohe Konzentrationen von Mikroorganismen zu erhalten, werden die Extrapolationen über eine längere Zeitdauer vorgenommen, und daher sind die extrapolierten Ergebnisse von erheblich geringerer Genauigkeit.

Ein allgemeiner Ansatz zum Bestimmen der Bakterienpopulation flüssiger Proben, insbesondere von Milchproben und Blutprodukt-Einheiten, basiert auf einer Messung der Impedanz über die Probe. Nachdem die Impedanz einer Probe im Allgemeinen mit wachsender Bakterienpopulation abfällt, bilden Impedanzmessungen über die Probe eine Grundlage zum Abschätzen der Bakterienpopulation in der Probe.

Für Impedanzmessungen in flüssigen Proben wird Elektroden in der Probe im Allgemeinen eher eine Wechselspannung als eine Gleichspannung zugeführt, nachdem eine Gleichspannung dazu neigt, eine schnelle, isolierende Polarisierung an der Schnittstelle zwischen der Flüssigkeit und den Elektroden zu verursachen. Wenn eine hochfrequente Wechselspannung von typischerweise 1 kHz oder mehr verwendet wird, wird die Wirkung einer solchen Polarisierung auf die gemessenen Impedanzen in hohem Maße reduziert. Bestehende Vorrichtungen zum Zählen von Mikroorganismen verwenden typischerweise eine Wechselspannung von 1 kHz oder 10 kHz.

Ein Nachteil vieler Bakterienwachstumstester besteht darin, daß sie invasiv und ungenau sind und lange Brutzeiten erfordern, um vernünftige Ergebnisse zu erzielen. Dies liegt hauptsächlich an der Tatsache, daß signifikante Impedanzänderungen, typischerweise einige Prozent, nur nach langen Brutzeiten erscheinen, wenn sehr hohe Bakterienkonzentrationen, typischerweise ab $10^6$ Bakterien pro $cm^3$ erreicht sind. Zusätzlich zu ihrer geringen Empfindlichkeit auf Änderungen in kleinen Bakterienpopulationen sind existierende Testgeräte extrem empfindlich hinsichtlich Temperaturänderungen in der Probe, welche sowohl die Wachstumsrate der Bakterienpopulation als auch, was noch wichtiger ist, den Betrag der gemessenen Impedanzen beeinflußt. Hinzu kommt, daß, nachdem eine Evaluierung von Populationen in der Vergangenheit im Allgemeinen auf einer Messung der Probe zu Beginn einer Bebrütung als einem Bezugspunkt basiert und zum Zeitpunkt des Testens kein Bezugspunkt zur Verfügung steht, eine hohe Erfassungsschwelle erforderlich ist, selbst wenn die Temperatur streng stabilisiert ist.

Ein Verfahren zum Erfassen von Bakterien in einer Weinprobe durch Messen der Impedanz der Probe ist in P.A. Henschke & D. Susan Thomas "Detection of Wine-Spoiling Jeasts by Electronic Methods", Journal of Applied Bacteriology 1988, Band 64, Seiten 123-133 beschrieben. Dieser Artikel schlägt die Verwendung einer ein sterilisiertes Medium als Referenz zu der getesteten Weinprobe enthaltenden Kammer vor. Der Bezug auf das sterilisierte Medium gleicht Änderungen in der Impedanz der Weinprobe auf Grund von physikalischen und chemischen Faktoren, die nicht auf ein mikrobielles Wachstum bezogen sind, aus. Um zwischen Weinhefen und Fremdhefen zu unterscheiden, beschäftigt sich dieser Artikel mit der Möglichkeit, das mikrobielle Wachstum in den Weinhefen unter Verwendung von Ethanol zu verzögern.

Vorrichtungen zum Überwachen von Bakterien, welche zur Aufnahme einer Mehrzahl von Flüssigkeitsproben angepaßt sind, wobei jede Probe mit einem Paar von Elektroden versehen ist, um Strom hindurchzuschicken, sind bekannt. Solche Vorrichtungen, welche typischerweise als Mehrsensor-Impedanzmessvorrichtungen bezeichnet werden, sind zur gleichzeitigen invasiven Überwachung einer Mehrzahl von flüssigen Proben hilfreich. Beispiele solcher Mehrprobentester sind in den US-Patentschriften Nrn. 3,890,201 und 4,072,578 beschrieben.

Die Vorrichtungen der US-Patente Nrn. 3,890,201 und 4,072578 weisen eine Mehrzahl von individuellen Kammern zur Aufnahme einer jeweiligen Mehrzahl separater beimpfter Proben auf. Die in dem US-Patent Nr. 4,072,578 beschriebene Vorrichtung weist auch eine computerisierte Einrichtung zum Steuern der Analyse des bakteriellen Wachstums in den separaten Kammern auf.

Eine Mehrkammertestvorrichtung auf der Grundlage der oben beschriebenen Prinzipien ist von Malthus Instruments Ltd., England, unter dem Handelsnamen Malthus 2000 Microbiology System erhältlich. Diese Vorrichtung mißt die Leitfähigkeit von bis zu 240 separaten Proben durch Anlegen einer Wechselspannung von 10 kHz über Elektroden, welche den Proben zugeordnet sind. Das mikrobielle Wachstum in jeder Probe wird durch Messen von Änderungen in der Leitfähigkeit einer Base, welche metabolisches $CO_2$ absorbiert, unter Verwendung eines computerisierten mikrobiologischen Analysegeräts erfaßt.

Es sollte jedoch verstanden werden, daß die vorgenannten Nachteile bestehender Einzelkammervorrichtungen, z.B. Invasivität und eine niedrige Empfindlichkeit, welche in einer geringen Genauigkeit resultieren und lange Testzeiten erfordern, ebenso für jede Kammer bestehender Mehrkammervorrichtungen gelten. Es sollte ebenfalls verstanden werden, daß keine der bestehenden Mehrkammervorrichtungen eine genaue Erfassung einer kleinen Anzahl von Mikroorganismen in Flüssigkeitsproben erlaubt.

Es ist zu verstehen, daß Techniken zur Analyse elektrischer Eigenschaften, insbesondere die Impedanzspektroskopie, in den letzten zehn Jahren beträchtlich verbessert worden sind. Daher können nun durch Entwerfen geeigneter empirischer Modelle des elektrischen Verhaltens einer Probe, d.h. Entwerfen von Ersatzschaltbildern der Probe, und durch Anwenden von in höherem Maße automatisierten, nicht kontaktierenden und genaueren Impedanzmeßtechniken eine endgültigere Evaluierung der elektrischen Eigenschaften von Proben möglich sein kann. Eine allgemeine Darstellung des Gebiets der Impedanzspektroskopie kann in J. Ross MacDonald "Impedance Spectroscopie, Emphasising Solid Materials and Systems", New York, 1987, gefunden werden. Bioanalytische Anwendungen der Impedanzspektroskopie können in Douglas B. Kell "The Principles and Potential of Electrical Admittance Spectroscopy: an Introduction", aus "Biosensors, Fundamentals and Applications", herausgegeben von Anthony P. F. Turner et al., Oxford University Press, 1987, gefunden werden.

Ein System zum Erfassen des Vorliegens von Mikroorganismen in einer Probe wird in den US-Patentschriften Nrn. 4,009,078, 4,200,493 und 4,246,343, jeweils unter dem Namen von Wilkens et al., beschrieben. Das beschriebene System beinhaltet eine Kultivierung von Mikroorganismen in einem Wachstumsmedium, welches sich in Kontakt mit einer Meßelektrode befindet, und eine Meßelektrode, während Änderungen im Potential zwischen den Elektroden gemessen werden.

Als nächstliegender Stand der Technik sind die Patentschrift EP0803728 und die Veröffentlichung "Microscale electronic detection of bacterial metabolism" von R. Gömez et al (Sensors and Actuators B 86 (2002), 198-208) zu nennnen. EP0803728 beschreibt eine nicht-invasive Impedanzmessung zur Überwachung mikrobiellen Wachstums. Der Artikel von Gómez beschreibt eine invasive Messung, die die Berechnung einer Warburg-Impedanz beinhaltet.

KURZFASSUNG DER ERFINDUNG

Die Aufgabe der vorliegenden Erfindung besteht darin, eine wesentlich verbesserte Vorrichtung und ein entsprechendes Verfahren zum Überwachen der Mikroorganismenpopulation in einer Probe unter Verwendung einer Impedanzanalyse zu schaffen.

Diese Aufgabe wird durch die Merkmale der unabhängigen Ansprüche gelöst. Vorteilhafte Ausführungsformen und Weiterbildungen der vorliegenden Erfindung bilden den Gegenstand der abhängigen Ansprüche.

Das Verfahren und die Vorrichtung der vorliegenden Erfindung erfordert kürzere Überwachungs- und Brutzeiten als vorbekannte Verfahren und Vorrichtungen. Außerdem ermöglicht die vorliegende Vorrichtung und das vorliegende Verfahren eine genauere Messung extrem kleiner Mikroorganismenpopulationen. Weiterhin sind das Verfahren und die Vorrichtung insbesondere zum nicht-invasiven Testen von flüssigen Proben in rohr- oder schlauchartigen Leitungen wie etwa in Zu- und Ableitungen von Behältern geeignet. Eine weitere Ausführungsform ist insbesondere zum nicht-invasiven Testen von Blutprodukt-Einheiten wie zum Beispiel Blutplättchen-Einheiten vorzugsweise in der Form von Beuteln geeignet.

Der vorliegenden Erfindung liegt die Grundidee zugrunde die gemessenen Impedanzen durch eine geeignete Schaltungsanordnung in vordefinierte reale und/oder imaginäre, d.h. komplexe, Komponenten zu zerlegen. Es hat sich herausgestellt, daß einige dieser Komponenten hinsichtlich Änderungen in der Bakerienpopulation in der Probe empfindlicher sind als die Gesamtimpedanz der Probe. Es erweist sich daher als vorteilhaft, die Bakterienpopulation in der Probe auf der Grundlage der stärker reagierenden Komponente zu analysieren.

Für die Anwendung an Milchproben und Plasmabehältern hat es sich als vorteilhaft erwiesen, die Bakterienpopulation auf der Grundlage einer Messung der Warburg-Impedanz zu analysieren. Dabei wird ein Element eines Ersatzschaltbildes, entweder von Milchproben oder von Blutprodukt-Einheiten, identifiziert und analysiert, wobei das Element jeweils besonders empfindlich hinsichtlich Änderungen der Bakterienkonzentration der Milch oder des Blutprodukt ist.

Weiter hat es sich als vorteilhaft erwiesen, in dem oben genannten Verfahren zum Evaluieren der Mikroorganismenpopulation in einer Flüssigkeitsprobe ein Ersatzschaltbild der Flüssigkeitsprobe zu definieren, den Betrag wenigstens eines Elements des Ersatzschaltbildes zu bestimmen, und die Anzahl von Mikroorganismen in der Probe auf der Grundlage des wenigstens einen bestimmten Elements zu evaluieren.

Es ist auch vorteilhaft, eine Wechselspannung mit einer Mehrzahl von Frequenzen über wenigstens einem Abschnitt der Probe anzulegen, die Impedanz über wenigstens den Abschnitt der Flüssigkeitsprobe in Reaktion auf wenigstens einige der Mehrzahl von Wechselfrequenzen zu messen, wenigstens ein Element eines elektrischen Ersatzschaltbildes der Probe auf der Grundlage der über wenigstens den Abschnitt gemessenen Impedanzen zu berechnen und die Mikroorganismenpopulation in der Probe auf der Grundlage des wenigstens einen berechneten Elements zu evaluieren.

Das Element beinhaltet eine warburg-Impedanz. Zusätzlich kann die Flüssigkeitsprobe für eine vorgewählte Zeitdauer vor der Messung der Impedanz bebrütet werden.

Eine entsprechende Vorrichtung zum Evaluieren der Mikroorganismenpopulation in einer Flüssigkeitsprobe weist einen zum Aufnehmen der Flüssigkeitsprobe geeigneten Behälter und einen Impedanzprozessor, welcher eine Wechselspannung bei einer Mehrzahl von Frequenzen über wenigstens einen Abschnitt der Flüssigkeitsprobe anlegt und die Impedanz über wenigstens den Abschnitt der Flüssigkeitsprobe in Reaktion auf wenigstens einige der Wechselfrequenzen mißt, auf, wobei der Impedanzprozessor eine Schaltungsanordnung zum Berechnen einer Warburg-Impedant der Probe auf der Grundlage der über wenigstens den Abschnitt gemessenen Impedanz und zum Evaluieren der Mikroorganismenpopulation in der Probe auf der Grundlage des wenigstens einen berechneten Elements aufweist.

Der Impedanzprozessor ist elektrisch in nicht-invasiver Weise mit der Flüssigkeitsprobe verbunden. In einer speziellen Bauform weist der Impedanzprozessor zwei Kondensatorplatten zum elektrischen nicht-invasiven Verbinden des Impedanzprozessors mit der Flüssigkeitsprobe auf. Die Flüssigkeitsprobe wiederum weist eine Mehrzahl von Seiten auf, und beide der zwei Kondensatorplatten sind operativ mit einer der Mehrzahl von Seiten verbunden.

Vorteilhafterweise weist die oben genannte Vorrichtung eine Grundplatte auf, und die Grundplatte und die zwei Kondensatorplatten sind im wesentlichen koplanar zueinander, wobei die Grundplatte die zwei Kondensatorplatten umgibt und trennt.

Ein entsprechender kapazitiver Sensor zur Verwendung beim Messen der Impedanz einer Flüssigkeitsprobe weist zwei Kondensatorplatten und eine Grundplatte auf, wobei die Grundplatte und die zwei Kondensatorplatten im wesentlichen koplanar zueinander sind und wobei die Grundplatte die zwei Kondensatorplatten umgibt und trennt. Der kapazitive Sensor kann dauerhaft an der Blutproben-Einheit angebracht sein.

Ein entsprechender Flüssigkeitsprobenhalter zur Verwendung bei einer Messung der Impedanz einer Flüssigkeitsprobe weist einen Behälter zum Aufnehmen der Flüssigkeitsprobe sowie einen kapazitiven Sensor mit zwei Kondensatorplatten und einer Grundplatte auf, wobei die Grundplatte und die zwei Kondensatorplatten im wesentlichen koplanar sind und wobei die Grundplatte die zwei Kondensatorplatten umgibt und trennt.

Die oben genannten Bauformen sind besonders geeignet zur Anwendung an einer Blutprodukt-Einheit in Beutelform. Die flüssige Probe kann eine Blutplättchen-Einheit aufweisen. Die Frequenzen können zwischen 10 Hz und 10 MHz liegen.

Gemäß der vorliegenden Erfindung wird das oben genannte Verfahren beispielsweise auf die Bestimmung der Mikroorganismenpopulation in einer in einem schlauch- oder röhrenförmigen Bauelement befindlichen flüssigen Probe angewendet.

Eine an diesen Anwendungsfall angepaßte Vorrichtung zum Bestimmen der Mikroorganismenpopulation in einer flüssigen Probe weist gemäß der Erfindung ein schlauchförmiges oder röhrenförmiges Bauelement zum Aufnehmen oder Leiten der flüssigen Probe darin; und einen Impedanzprozessor, welcher eine Wechselfrequenz bei einer Mehrzahl von Frequenzen über wenigstens einen Abschnitt der flüssigen Probe anlegt und die Impedanz über wenigstens den Abschnitt der flüssigen Probe in Reaktion auf wenigstens einige der Wechselfrequenzen mißt, auf, wobei der Impedanzprozessor eine Mehrzahl von Schaltungsanordnungen zum Berechnen wenigstens eines Elements eines elektrischen Ersatzschaltbildes der Probe auf der Grundlage der über wenigstens den Abschnitt gemessenen Impedanzen und zum Bestimmen der Mikroorganismenpopulation in der Probe auf der Grundlage des wenigstens einen berechneten Elements aufweist.

Bei der erfindungsgemäßen Vorrichtung weist der Impedanzprozessor vorzugsweise zwei Kondensatorelektroden zum elektrischen nicht-invasiven Verbinden des Impedanzprozessors mit der flüssigen Probe auf, die um die flüssige Probe herum mit einem vorbestimmten Abstand in axialer Richtung des schlauchförmigen oder röhrenförmigen Bauelements voneinander angeordnet sind.

Ein kapazitiver Sensor zur Verwendung beim Messen der Impendanz einer in einem schlauchförmigen oder röhrenförmigen Bauelement befindlichen flüssigen Probe weist gemäß der Erfindung zwei Elektroden auf, die unter einem vorbestimmten Abstand in axialer Richtung des schlauchförmigen oder röhrenförmigen Bauelements voneinander um die flüssige Probe herum angeordnet sind.

KURZE BESCHREIBUNG DER ZEICHNUNG

Die vorliegende Erfindung wird aus der nachfolgenden genauen Beschreibung der bevorzugten Ausführungsformen der vorliegenden Erfindung ersichtlich, welche in Verbindung mit den beiliegenden Zeichnungen beschrieben werden, in welchen:

Fig. 1 eine schematische Darstellung eines elektrischen Ersatzschaltbildes von Milch ist;

Fig. 2 eine vereinfachte Darstellung eines erfindungsgemäßen kapazitiven Sensors zur Verwendung an einer Blutprodukt-Einheit ist;

Fig. 3 eine vereinfachte Darstellung der Vorrichtung von Fig. 2 bei Verwendung mit einer Blutprodukt-Einheit ist;

Fig. 4 eine Seitenansicht der Vorrichtung von Fig. 3 ist;

Fig. 5 eine vereinfachte Darstellung eines Systems zur Messung einer Blutprodukt-Einheit unter Einschluß der Vorrichtung von Fign. 3 und 4 ist;

Fig. 6 eine schematische Darstellung eines elektrischen Ersatzschaltbildes einer Blutprodukt-Einheit zusammen mit der Elektrodenanordnung von Fig. 2 ist;

Fig. 7 eine vereinfachte Darstellung einer Vorrichtung zur Messung einer bakteriellen Belastung in einer flüssigen Probe gemäß der vorliegenden Erfindung ist;

GENAUE BESCHREIBUNG DER AUSFÜHRUNGSFORMEN

Es hat sich herausgestellt, daß empfindlichere Messungen erhalten werden können, wenn nicht die Gesamtimpedanz der Proben, sondern nur eine bestimmte Komponente der Probenimpedanzen durch den Impedanzprozessor gemessen wird. Um eine gewünschte Impedanzkomponente zu synthetisieren, ist es erforderlich, ein elektrisches Ersatzschaltbild der im Test befindlichen Probe zu konstruieren, in welchem die gewünschte Impedanz als ein unterscheidbares Schaltungselement enthalten ist.

Nachdem die Impedanz des Ersatzschaltbildes definitionsgemäß der Impedanz der im Test befindlichen Probe äquivalent ist, ist es möglich, einen Satz von nichtlinearen Gleichungen in Abhängigkeit von dem Probenmaterial durch Messen der Probenimpedanz bei einer Mehrzahl von Wechselfrequenzen aufzustellen. Die Anzahl der erforderlichen Impedanzmessungen entspricht der Anzahl der Unbekannten in dem Ersatzschaltbild. Vorzugsweise werden eine Mehrzahl von Wechselfrequenzen in dem Bereich von etwa 10 Hz bis 10 MHz verwendet, während bei vorbekannten Vorrichtungen, die eine konstante Frequenz verwenden, typischerweise 1 kHz oder 10 kHz verwendet werden.

Für eine Übersicht über Ersatzschaltbilder in der Impedanzspektroskopie sei auf J. Ross MacDonald, "Impedance Spectroscopie, Emphasising Solid Materials and Systems", New York, 1987 verwiesen. Bezüglich Ersatzschaltbildern für Mikroorganismenwachstum sei auf J. C. S. Richard, A. C. Jason, G. Hobbs, D. M. Gibson und R. H. Christie, "Electronic Measurement of Bacterial Growth", in "Journal of Physics and Electronics Scientific Instruments", Band II, 1978, verwiesen.

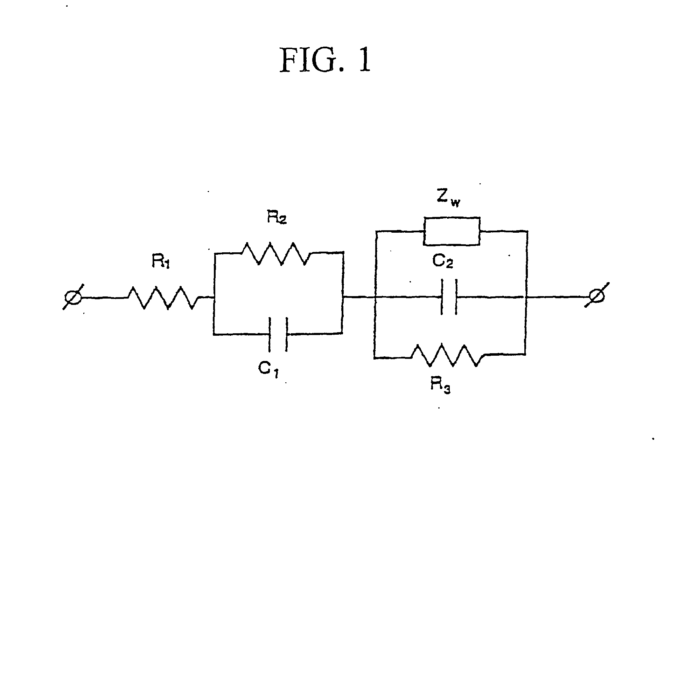

Fig. 1 stellt ein bevorzugtes Ersatzschaltbild zur invasiven Messung von Milchproben dar. Wie in Fig. 1 gesehen werden kann, beinhaltet das Ersatzschaltbild drei Widerstände $R_1$, $R_2$ und $R_3$, zwei Kondensatoren $C_1$ und $C_2$ und eine als $Z_W$ bezeichnete Komponente, die als Warburg-Impedanz bekannt ist. Die Warburg-Impedanz, wie sie im Stand der Technik bekannt ist, ist durch die nachfolgende Gleichung gegeben:

$$Z_W = W\,(1 - j)/(2\pi f)^{1/2},$$

wobei:

$Z_W$ die Warburg-Impedanz ist;

W die Warburg-Variable ist;

j die imaginäre Impedanznotation ist; und

f die verwendete Wechselfrequenz ist.

Es hat sich herausgestellt, daß die Warburg-Impendanz wenigstens zehnmal so empfindlich auf Änderungen in der Anzahl von Bakterien in Milch reagiert als die Gesamtimpedanz der Schaltung.

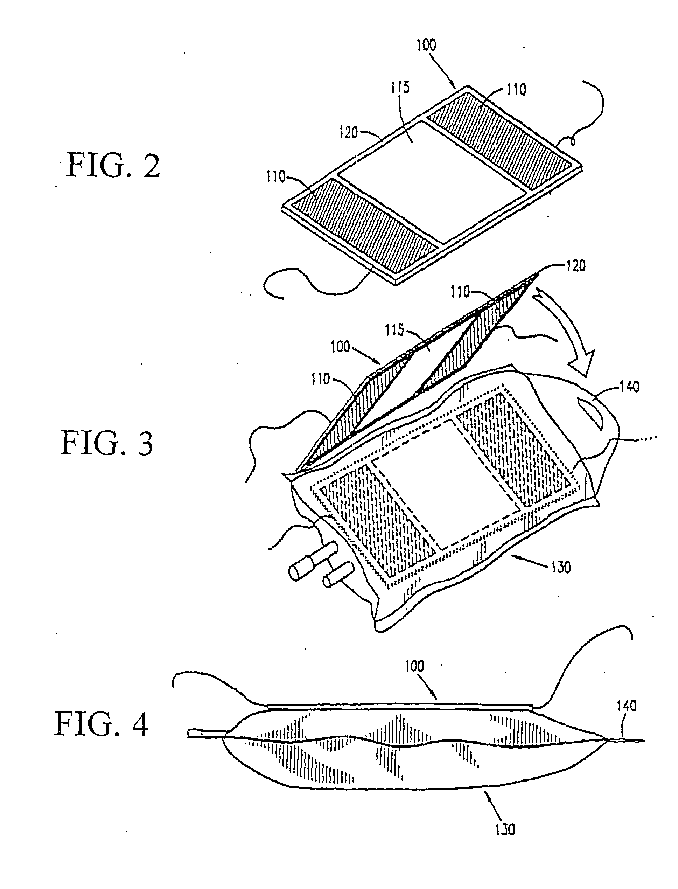

Mit Bezug auf Fig. 2 wird nun ein kapazitiver Sensor zur Verwendung im Zusammenhang mit einer Blutprodukt-Einheit beschrieben werden, wobei die Vorrichtung von Fig. 2 einen kapazitven Sensor 100 aufweist.

Der kapazitve Sensor 100 weist vorzugsweise zwei Kondensatorplatten 110 auf. Die zwei Kondensatorplatten 110 sind vorzugsweise auf der gleichen Seite eines vorzugsweise flachen Substrats 115 angeordnet. Der kapazitive Sensor 100 weist ebenfalls vorzugsweise eine Grundplatte 120 auf. Vorzugsweise sind die zwei Kondensatorplatten 110 und die Grundplatte 120 derart angeordnet, daß die Grundplatte 120 die zwei Kondensatorplatten 110 umgibt und die eine der zwei Kondensatorplatten 110 von der anderen Kondensatorplatte 110 trennt.

Die Grundplatte 120 dient dazu, den Einfluß der Oberfläche eines Beutels oder anderen Behälters auszuschließen, wenn eine in dem Beutel enthaltene Blutprodukt-Einheit gemessen wird. Es wird angenommen, daß bei Verwendung der Vorrichtung von Fig. 2 eine optimale Meßempfindlichkeit erreicht wird, wenn sich jede der Kondensatorplatten 110 in einer im wesentlichen rechteckigen Konfiguration befindet, jede der Kondensatorplatten 110 von im wesentlichen gleicher Breite ist und der Abstand zwischen den einander zugewandten Kanten der zwei Kondensatorplatten 110 näherungsweise gleich der Summe der Breiten der zwei Kondensatorplatten 110 ist, wie in Fig. 2 gesehen werden kann.

Die Vorrichtung von Fig. 2 kann unter Verwendung eines beliebigen einer Anzahl von Verfahren hergestellt werden, die im Stand der Technik wohlbekannt sind. Beispielsweise kann eine ungeätzte Computerleiterplatte verwendet und die Leiterplatte dann in wohlbekannter Weise geätzt werden, um die Vorrichtung von Fig. 2 herzustellen.

Anhand von Fig. 3 wird nun eine Verwendung der Vorrichtung von Fig. 2 an einer Blutprodukt-Einheit beschrieben werden.

Die Vorrichtung von Fig. 3 weist vorzugsweise den kapazitiven Sensor 100 von Fig. 2 und ebenfalls vorzugsweise eine Blutprodukt-Einheit 130 auf. Die Blutprodukt-Einheit 130 weist üblicherweise einen Sack 140 auf, welcher typischerweise aus einem Kunststoff hergestellt ist. In Fig. 3 ist der kapazitive Sensor 100 so dargestellt, daß er auf dem Sack 140 angeordnet ist.

Es wird angenommen, daß beide der Kondensatorplatten 110 auf einer Seite des Sacks 140 montiert sind und sich im allgemeinen im Kontakt damit befinden. Es wird ebenfalls angenommen, daß die in Fig. 3 gezeigte Konfiguration vorzugsweise angewendet wird. Andererseits sind auch andere Konfigurationen möglich, wie etwa eine Anordnung derart, daß die Kondensatorplatten 110 auf entgegengesetzten Seiten des Sacks 140 oder auch innerhalb des Sacks 140 angeordnet sind.

Zusätzlich wird nun auf Fig. 4 Bezug genommen, welche eine Seitenansicht ist, die zeigt, daß der kapazitive Sensor 100 auch dauerhaft an dem Sack 140 angebracht sein kann. In diesem Fall wäre der kapazitive Sensor 100 typischerweise wegwerfbar.

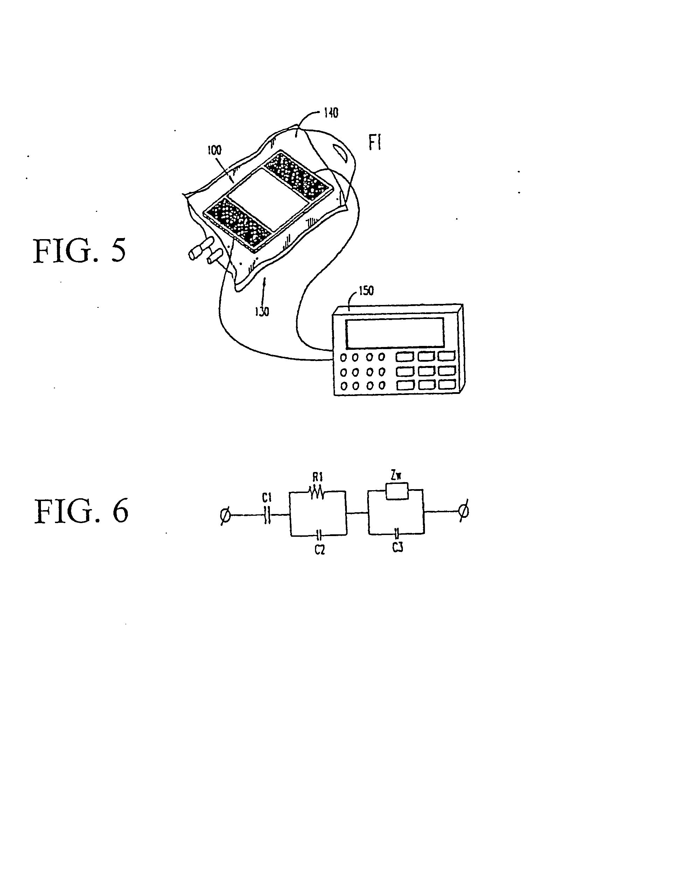

Mit Bezug auf Fig. 5 wird nun ein System zum Messen einer Blutprodukt-Einheit beschrieben werden, welches die Vorrichtungen von Fign. 3 und 4 beinhaltet. Das System von Fig. 5 weist den kapazitiven Sensor 100 und die Blutprodukt-Einheit 130 einschließlich des Sacks 140 auf. Das System von Fig. 5 weist ebenfalls einen Impedanzprozessor 150 auf, der operativ mit dem kapazitiven Sensor 100 verbunden ist. Die Impedanzmeßeinheit 150 kann jede geeignete Impedanzmeßeinheit sein, wie etwa ein HP 4284A, der von Hewlett-Packard käuflich zu erwerben ist.

Typischerweise ist die Verbindung zwischen dem Impedanzprozessor 150 und dem kapazitiven Sensor 100 unter Verwendung einer Mehrzahl von Drähten ausgebildet. Typischerweise ist ein Draht mit jeder der Kondensatorplatten 110 verbunden, während ein Draht mit der Grundplatte 120 verbunden ist; es kann jedoch auch andere Anordnungen der Befestigung geben, die für den besonderen Impedanzprozessor 150 geeignet sind.

Nun wird die Betriebsweise des Systems von Fig. 5 kurz beschrieben werden. Der kapazitive Sensor 100 ist in Kontakt mit der Blutprodukt-Einheit 130 angeordnet. Der Impedanzprozessor 150 weist eine Funktion auf, die Impedanz des Systems, welches den kapazitiven Sensor 100 und die Blutprodukt-Einheit 130 aufweist, bei einer Mehrzahl von Wechselfrequenzen zu messen, wie zuvor beschrieben, und ist dann tätig, um eine Ersatzschaltbildanalyse auszuführen, wie zuvor beschrieben. In Fig. 6 ist zusätzlich eine schematische Darstellung eines elektrischen Ersatzschaltbildes einer Blutprodukt-Einheit zusammen mit der Elektrodenanordnung von Fig. 2 gezeigt. Die Schaltung von Fig. 6 hat sich zur Verwendung bei der Analyse von Blutprodukt-Einheiten als geeignet erwiesen.

Die Schaltung von Fig. 6 beinhaltet drei Kondensatoren $C_1$, $C_2$ und $C_3$, einen Widerstand $R_1$ und eine Warburg-Impedanz $Z_W$.

Der Impedanzprozessor 150 weist eine Funktion auf, die Warburg-Impedanz der Schaltung von Fig. 6 zu berechnen. Es hat sich herausgestellt, daß die berechnete Warburg-Impedanz für die Schaltung von Fig. 6 gut mit einem bakteriellen Wachstum in der Blutprodukt-Einheit 130 korreliert, wobei die Warburg-Impedanz bei höheren Graden bakterieller Kontamination signifikant abfällt.

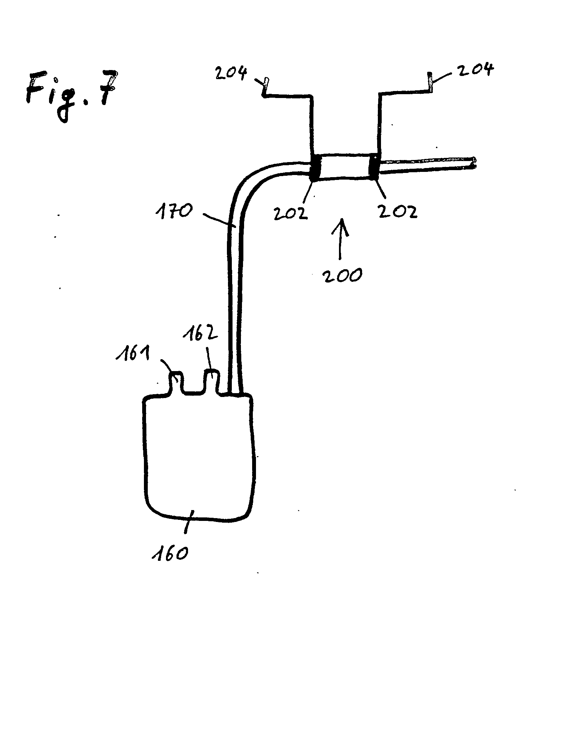

Fig. 7 ist eine vereinfachte Darstellung eines Grundprinzips einer Vorrichtung zum Messen einer bakteriellen Belastung in einer flüssigen Probe an einem schlauch- oder röhrenförmigen Bauteil gemäß der vorliegenden Erfindung. Ein Behälter 160 wie etwa ein Beutel, der eine Flüssigkeit enthält, wie etwa eine Blutprodukt-Einheit, weist zwei Stutzen 161 und 162 auf, die beispielsweise zum Befüllen des Behälters verwendet werden. Grundsätzlich kann der Behälter 160 jede andere Form annehmen und kann auch mehr als zwei Stutzen, nur einen Stutzen oder überhaupt keinen Stutzen aufweisen. An dem Behälter ist ein schlauch- bzw. röhrenförmiges Bauteil 170 (nachstehend als Schlauch 170 bezeichnet) verbunden, durch welchen bzw. in welchem eine Flüssigkeit in den Behälter oder aus dem Behälter strömen kann oder auch bewegungslos stehen kann. An dem Schlauch 170 ist ein kapazitiver Sensor 200 angebracht. Der kapazitive Sensor 200 besteht im wesentlichen aus zwei Elektroden 202, die mit einem vorbestimmten Abstand voneinander in Längsrichtung des Schlauchs 170 so angeordnet sind, daß sie jeweils die in dem Schlauch 170 befindliche Flüssigkeit umgeben.

**Patentansprüche**

1. Verfahren zum Bestimmen der Mikroorganismenpopulation in einer flüssigen Probe, welches aufweist:

    Anlegen von einer Mehrzahl von Wechselspannungen einer Mehrzahl von Frequenzen über wenigstens einen Abschnitt der flüssigen Probe;
    Messen der Impedanz über den wenigstens einen Abschnitt der flüssigen Probe als Reaktion auf wenigstens einige der Mehrzahl von Frequenzen;
    Berechnen wenigstens einer Impedanz eines Elements eines elektrischen Ersatzschaltkreises der flüssigen Probe auf der Grundlage der über den wenigstens einen Abschnitt gemessenen Impedanz, wobei das wenigstens eine berechnete Element eine Warburg-Impedanz aufweist; und
    Bestimmen der Mikroorganismenpopulation in der flüssigen Probe auf der Grundlage der Impedanz des wenigstens einen berechneten Elements

    **dadurch gekennzeichnet, daß**
    das Messen der Impedanz nicht-invasiv durchgeführt wird.

2. Verfahren gemäß Anspruch 1, **dadurch gekennzeichnet, daß** die Frequenzen zwischen 10 Hz und 10 MHz liegen.

3. Verfahren gemäß Anspruch 1, **dadurch gekennzeichnet, daß** es weiter eine Bebrütung der flüssigen Probe für eine vorbestimmte Zeitdauer vor der Messung der Impedanz beinhaltet.

4. Verfahren gemäß einem der Ansprüche 1 bis 3, **dadurch gekennzeichnet, daß** sich die flüssige Probe in einem schlauchförmigen oder röhrenförmigen Bauelement (170) befindet.

5. Vorrichtung zum Bestimmen der Mikroorganismenpopulation in einer flüssigen Probe, welche aufweist:

    einen Behälter zum Aufnehmen oder Leiten der flüssigen Probe darin; und
    einen Impedanzprozessor, welcher eingerichtet ist um eine Mehrzahl von Wechselspannungen einer Mehrzahl von Frequenzen über wenigstens einen Abschnitt der flüssigen Probe anzulegen und die Impedanz über den wenigstens einen Abschnitt der flüssigen Probe als Reaktion auf wenigstens einige der Mehrzahl von Frequenzen zu messen, wobei

    der Impedanzprozessor eine Mehrzahl von Schaltungsanordnungen zum Berechnen einer Impedanz wenigstens eines Elements eines elektrischen Ersatzschaltkreises der flüssigen Probe auf der Grundlage der über wenigstens den Abschnitt gemessenen Impedanz und zum Bestimmen der Mikroorganismenpopulation in der flüssigen Probe auf der Grundlage der Impedanz des wenigstens einen berechneten Elements aufweist, und das wenigsten eine berechnete Element eine Warburg-Impedanz aufweist,
    **dadurch gekennzeichnet, daß** der Behälter so eingerichtet ist, daß
    der Impedanzprozessor mit der flüssigen Probe nicht-invasiv verbunden ist.

**6.** Vorrichtung gemäß Anspruch 5, *dadurch gekennzeichnet, daß* die Wechselfrequenzen zwischen 10 Hz und 10 MHz liegen.

**7.** Vorrichtung gemäß Anspruch 5, *dadurch gekennzeichnet, daß* der Impedanzprozessor zwei Kondensatorelektroden (202) zum elektrischen nicht-invasiven Verbinden des Impedanzprozessors mit der flüssigen Probe aufweist.

**8.** Vorrichtung gemäß Anspruch 7, *dadurch gekennzeichnet, daß* die flüssige Probe eine Mehrzahl von Seiten aufweist und beide der zwei Kondensatorplatten operativ mit einer der Mehrzahl von Seiten verbunden sind.

**9.** Vorrichtung gemäß Anspruch 7, *dadurch gekennzeichnet, daß* sie weiterhin eine Grundplatte aufweist, wobei die Grundplatte und die zwei Kondensatorplatten im wesentlichen koplanar zueinander sind und die Grundplatte die zwei Kondensatorplatten umgibt und trennt.

**10.** Vorrichtung gemäß einem der Ansprüche 7 bis 9, *dadurch gekennzeichnet, daß* der Behälter ein schlauchförmiges oder röhrenförmiges Bauelement (170) ist.

**11.** Vorrichtung gemäß Anspruch 7, *dadurch gekennzeichnet,* **daß** die Kondensatorelektroden (202) um die flüssige Probe herum mit einem vorbestimmten Abstand in axialer Richtung des schlauchförmigen oder röhrenförmigen Bauelements (170) voneinander angeordnet sind.

**Claims**

**1.** Method for determining the micro-organism population in a liquid sample, which comprises:

applying a plurality of AC voltages at a plurality of frequencies across at least one section of the liquid sample;
measuring the impedance across the at least one section of the liquid sample in response to at least some of the plurality of frequencies;
calculating at least one impedance for an element of an electrical equivalent circuit for the liquid sample on the basis of the impedance measured across the at least one section, with the at least one calculated element having a Warburg impedance; and
determining the micro-organism population in the liquid sample on the basis of the impedance of the at least one calculated element,

**characterized in that**
the impedance is measured non-invasively.

**2.** Method according to Claim 1, **characterized in that** the frequencies are between 10 Hz and 10 MHz.

**3.** Method according to Claim 1, **characterized in that** it also comprises incubating the liquid sample for a predetermined period before measurement of the impedance.

**4.** Method according to one of Claims 1 to 3, **characterized in that** the liquid sample is in a hose-like or tubular component (170).

**5.** Apparatus for determining the micro-organism population in a liquid sample, which comprises:

a container for holding or routing the liquid sample therein; and
an impedance processor which is set up to apply a plurality of AC voltages at a plurality of frequencies across at least one section of the liquid sample and to measure the impedance across the at least one section of the liquid sample in response to at least some of the plurality of frequencies, where

the impedance processor has a plurality of circuit arrangements for calculating an impedance for at least one element of an electrical equivalent circuit for the liquid sample on the basis of the impedance measured across at least the section and for determining the micro-organism population in the liquid sample on the basis of the impedance of the at least one calculated element, and the at least one calculated element has a Warburg impedance,
**characterized in that** the container is set up such that the impedance processor is non-invasively connected to the liquid sample.

**6.** Apparatus according to Claim 5, **characterized in that** the AC frequencies are between 10 Hz and 10 MHz.

**7.** Apparatus according to Claim 5, **characterized in that** the impedance processor has two capacitor electrodes (202) for electrically non-invasively connecting the impedance processor to the liquid sample.

**8.** Apparatus according to Claim 7, **characterized in that** the liquid sample has a plurality of sides, and both of the two capacitor plates are operatively connected to one of the plurality of sides.

**9.** Apparatus according to Claim 7, **characterized in that** it also has a baseplate, with the baseplate and the two capacitor plates being essentially coplanar with respect to one another, and the baseplate surrounding and isolating the two capacitor plates.

**10.** Apparatus according to one of Claims 7 to 9, **characterized in that** the container is a hose-like or tubular component (170).

**11.** Apparatus according to Claim 7, **characterized in that** the capacitor electrodes (202) are arranged around the liquid sample at a predetermined distance from one another in the axial direction of the hose-like or tubular component (170).

**Revendications**

**1.** Procédé pour déterminer des populations de micro-organismes dans un échantillon liquide, comportant :

l'application d'une pluralité de tensions alternatives sur au moins une partie de l'échantillon liquide ;
la mesure de l'impédance sur au moins la partie de l'échantillon liquide en tant que réaction à au moins quelques unes parmi la pluralité de fréquences ;
le calcul d'au moins une impédance d'un élément d'un circuit de commutation électrique de réserve de l'échantillon liquide, sur la base de l'impédance mesurée sur au moins la partie, le au moins un élément calculé présentant une impédance de Warburg ;
et détermination de la population de micro-organismes dans l'échantillon liquide, sur la base de l'impédance du au moins un élément calculé, **caractérisé en ce que** la mesure de l'impédance est réalisée de façon non invasive.

**2.** Procédé selon la revendication 1, **caractérisé en ce que** les fréquences se situent entre 10 Hz et 10 MHz.

**3.** Procédé selon la revendication 1, **caractérisé en ce qu'**il comporte par ailleurs une incubation de l'échantillon liquide sur une période déterminée, avant la mesure de l'impédance.

**4.** Procédé selon l'une quelconque des revendications 1 à 3, **caractérisé en ce que** l'échantillon liquide se trouve dans un élément constitutif (170) en forme de boyau ou de tube.

**5.** Dispositif pour déterminer des populations de micro-organismes dans un échantillon liquide, comportant :

un réservoir pour réceptionner ou pour diriger l'échantillon liquide dans son intérieur; et
un processeur d'impédance, qui est installé pour appliquer une pluralité de tensions alternatives d'une pluralité de fréquences sur au moins une partie de l'échantillon liquide et pour mesurer l'impédance sur la au moins une partie de l'échantillon liquide, en tant que réaction à au moins quelques unes parmi la pluralité de fréquences,

le processeur d'impédance comportant une pluralité d'agencements de circuits, pour calculer l'impédance d'au moins un élément d'un circuit de commutation électrique de réserve de l'échantillon liquide, sur la base de l'impédance mesurée sur la au moins une partie, et pour déterminer la population de micro-organismes dans l'échantillon liquide, sur la base de l'impédance du au moins un élément calculé, et le au moins un élément calculé présentant une impédance de Warburg, **caractérisé en ce que** le réservoir est installé de sorte à ce que le processeur d'impédance soit relié de façon non invasive avec l'échantillon liquide.

**6.** Dispositif selon la revendication 5, **caractérisé en ce que** les fréquences alternatives se situent entre 10 Hz et 10 MHz.

**7.** Dispositif selon la revendication 5, **caractérisé en ce que** le processeur d'impédance comporte deux électrodes de condensateur (202), pour la liaison électrique non invasive du processeur d'impédance avec l'échantillon liquide.

**8.** Dispositif selon la revendication 7, **caractérisé en ce que** l'échantillon liquide comporte une pluralité de faces et **en ce que** les deux parmi les deux plaques de condensateur sont reliées avec une pluralité de faces.

**9.** Dispositif selon la revendication 7, **caractérisé en ce qu'**il comporte par ailleurs une plaque de base, la plaque de base et les deux plaques de condensateur étant sensiblement coplanaires, l'une par rapport à l'autre, et **en ce que** la plaque de base entourant et séparant les deux plaques de condensateur.

**10.** Dispositif selon l'une quelconque des revendications 7 à 9, **caractérisé en ce que** le réservoir est un élément constitutif en forme de boyau ou de tube.

**11.** Dispositif selon la revendication 7, **caractérisé en ce que** les électrodes de condensateur (202) sont placées autour de l'échantillon liquide à un écart déterminé en direction axiale de l'élément constitutif (170) en forme de boyau ou de tube.

# FIG. 1

FIG. 2

FIG. 3

FIG. 4

FIG. 5

FIG. 6

Fig.7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4072578 A
- US 4009078 A
- US 4200493 A
- US 4246343 A
- EP 0803728 A

**In der Beschreibung aufgeführte Nicht-Patentliteratur**

- **P.A. HENSCHKE ; D. SUSAN THOMAS.** Detection of Wine-Spoiling Jeasts by Electronic Methods. *Journal of Applied Bacteriology,* 1988, vol. 64, 123-133
- **J. ROSS MACDONALD.** *Impedance Spectroscopie, Emphasising Solid Materials and Systems,* 1987
- Biosensors, Fundamentals and Applications. **DOUGLAS B. KELL ; ANTHONY P. F. TURNER et al.** The Principles and Potential of Electrical Admittance Spectroscopy: an Introduction. Oxford University Press, 1987
- **R. GÖMEZ ; SENSORS ; ACTUATORS B et al.** *Microscale electronic detection of bacterial metabolism,* 2002, vol. 86, 198-208
- **J. C. S. RICHARD ; A. C. JASON ; G. HOBBS ; D. M. GIBSON ; R. H. CHRISTIE.** *Electronic Measurement of Bacterial Growth'', in ''Journal of Physics and Electronics Scientific Instruments,* 1978, vol. II